# EUROPÄISCHE PATENTSCHRIFT

(11) **EP 3 397 118 B1**
(45) Veröffentlichungstag und Bekanntmachung des Hinweises auf die Patenterteilung: **19.01.2022**
(21) Anmeldenummer: 16820311.5
(22) Anmeldetag: 30.12.2016
(51) Int. Cl.: A47C 20/04, A47C 31/00, A61B 5/00

(54) **SCHLAF- ODER RUHEMÖBEL**
SLEEPING OR RECLINING FURNITURE
MEUBLE DE COUCHAGE OU DE REPOS

(30) Priorität: 30.12.2015 DE 202015107148 U; 24.05.2016 DE 102016109524; 06.07.2016 DE 202016103605 U
(43) Veröffentlichungstag der Anmeldung: 07.11.2018
(73) Patentinhaber: DewertOkin Technology Group Co., Ltd., Jiaxing City, Zhejiang Province (CN)
(72) Erfinder: GEHRKE, Karsten, 32457 Porta Westfalica (DE); HILLE, Armin, 33659 Bielefeld (DE); LOLEY, Steffen, 49082 Osnabrück (DE); TEWS, Alexander, 33607 Bielefeld (DE)
(74) Vertreter: Kleine, Hubertus
(86) Internationale Anmeldenummer: PCT/EP2016/082911
(87) Internationale Veröffentlichungsnummer: WO 2017/114945

(56) Entgegenhaltungen:
- WO-A1-2013/173640
- JP-A- 2005 177 471
- JP-A- 2005 253 957
- US-A1- 2010 101 022
- US-A1- 2014 266 733

## Beschreibung

Die Erfindung betrifft ein Schlaf- oder Ruhemöbel, insbesondere ein Bett, mit einem elektromotorischen Möbelantrieb.

Elektromotorische Möbelantriebe in Schlaf- oder Ruhemöbeln, beispielsweise Betten, Schlafsofas oder Ruhesesseln, ermöglichen auf einfache Weise, eine geeignete Schlaf- oder Ruheposition einzustellen. Bei Betten kann beispielsweise ein Rücken- oder Beinteil gegenüber einem Mittelteil des Betts angehoben oder abgesenkt werden. Mit elektromotorischen Antrieben ausgestattete Betten motivieren das Aufsuchen und Einstellen einer bestmöglichen Schlafposition, wohingegen dieses bei nicht oder nur manuell verstellbaren Betten aus Gründen der Bequemlichkeit oder aufgrund von mangelnder körperlicher Bewegungsfähigkeit unterbleibt.

Beispiele von solchen Schlaf- oder Ruhemöbeln sind aus WO2013/173640A1, JP2005177471A, JP2005253957A, US2010/101022A1 und US2014/266733A1 bekannt.

Im klinischen Bereich sind Überwachungsgeräte bekannt, die Atmung und/oder Herztätigkeit eines Patienten im Schlaf überwachen, um bei bedenklichen Herzfunktions- und Kreislaufparametern eingreifen zu können.

Inzwischen sind Vorrichtungen zur Überwachung des Schlafzustands anhand von physiologischen Parametern auch für nichtklinische Zwecke im Handel erhältlich. Diese Geräte, die beispielsweise auf einen Nachtisch gestellt werden, erfassen Geräusche und/oder Bewegungszustände während des Schlafs mithilfe von Mikrofonen und/oder Kameras. Aus den erfassten Informationen wird ein Schlafzustand abgeleitet, dessen zeitlicher Verlauf aufgezeichnet wird. Der aufgezeichnete Schlafverlauf kann nachträglich abgerufen und ausgewertet werden. Er kann Aufschluss darüber geben, wie tief und erholsam der Schlaf gewesen ist.

Neben Systemen, die Kamera und/oder Mikrofon einsetzen, ist ein sensorbasiertes System bekannt, bei dem ein druckempfindlicher Sensorstreifen über die Matratze gelegt wird und bei dem dieser Sensorstreifen mit einem Mobiltelefon (Smartphone) verbunden wird, das die Sensordaten aufzeichnet. Aus den Sensordaten wird unter anderem eine Herzfrequenz und eine Atemfrequenz abgeleitet.

Nachteilig an den genannten nichtklinischen Systemen ist, dass die Zuverlässigkeit der Erkennung stark von der korrekten Positionierung der Überwachungsgeräte auf einen Nachtisch bzw. auf oder an der Matratze abhängt. Die Zuverlässigkeit und auch der Benutzungskomfort dieser Geräte sind dadurch eingeschränkt.

Es ist eine Aufgabe der vorliegenden Erfindung, ein Schlaf- oder Ruhemöbel und einen elektromotorischen Möbelantrieb der eingangs genannten Art zu schaffen, die den Benutzer weiter unterstützen, einen tiefen und erholsamen Schlaf zu finden.

Diese Aufgabe wird durch ein Schlaf- bzw. Ruhemöbel mit einem elektromotorischen Möbelantrieb mit den jeweiligen Merkmalen der unabhängigen Ansprüche gelöst. Vorteilhafte Ausgestaltungen und Weiterbildungen sind in den abhängigen Ansprüchen angegeben.

Der mechanisch mit dem Möbel gekoppelte und elektrisch in das System des elektromotorischen Antriebs eingebundene Sensor führt zu einem benutzerfreundlichen und im Hinblick auf eine Fehlbedienung, insbesondere Fehlpositionierung, fehlersicheren System zur Erfassung der physiologischen Parameter. Erfasste physiologische Parameter sind eine Herzfrequenz, eine Atemfrequenz und/oder ein Bewegungszustand der das Schlaf- oder Ruhemöbel benutzenden Person.

Erfindungsgemäß weist der elektromotorische Möbelantrieb eine Steuereinrichtung zur Ansteuerung der Verstellmotoren auf, wobei die Auswerteeinheit mit der Steuereinrichtung gekoppelt ist oder in die Steuereinrichtung integriert ist. Auf diese Weise können Komponenten der Steuereinrichtung, die bei dem elektromotorischen Möbelantrieb bereits vorhanden sind, auch für die Auswerteeinheit genutzt werden, beispielsweise eine Stromversorgungseinheit, Kommunikationseinrichtungen und/oder ein Gehäuse einschließlich der Anschlussmöglichkeiten. Zudem vereinfacht sich eine Verkabelung des Sensors am oder unter dem Bett, wenn die vorhandene Struktur des elektromotorischen Möbelantriebs verwendet wird.

Erfindungsgemäß weist einen elektromotorischen Möbelantrieb der mindestens eine Sensor einen piezoelektrisch arbeitenden Schall- bzw. Schwingungsaufnehmer auf. Ein solcher Sensor arbeitet robust und ist geeignet, sowohl Vibrationen (Körperschall), als auch Luftschall aufzunehmen, wobei konstruktiv festgelegt werden kann, in welchem Verhältnis diese unterschiedlichen Schallarten erfasst werden. Weiter können auch mehrere Schall- bzw. Schwingungsaufnehmer kombiniert werden, wobei beispielsweise ein piezoelektrisch und elektromagnetisch arbeitender Schall- bzw. Schwingungsaufnehmer an gleicher Position oder an verschiedenen Positionen angeordnet sind. Bevorzugt weist der Sensor ein Sensorgehäuse mit einer Montagefläche auf, auf der eine Erhöhung ausgebildet ist, wobei ein Schall- bzw. Schwingungsaufnehmer des Sensors im Inneren des Sensorgehäuses im Bereich der Erhöhung angeordnet ist. Auf diese Weise kann eine besonders gute Vibrationsübertagung von der Möbelkomponente, auf der der Sensor montiert ist, auf den Schall- bzw. Schwingungsaufnehmer erreicht werden. Die Montagefläche, die z.B. Teil einer Montageplatte mit Befestigungslöchern sein kann, kann unkompliziert an einer ebenen Bettkomponente befestigt werden. Eine solche ebene Bettkomponente ist beispielsweise ein plattenförmiges Rückenteil eines Stützelements eines Betts. Zur Montage an einem Lattenrost als Stützelement ist bevorzugt ein Montageadapter vorgesehen, der mit dem Sensorgehäuse verbunden wird um eine Latte des Lattenrosts zur Montage des Sensors an dieser Latte zu umgreifen.

Erfindungsgemäß weist die Auswerteeinheit einen Filter, insbesondere einen Tief- oder Bandpassfilter zur Signalverarbeitung auf. Alternativ oder zusätzlich kann auch im Sensorgehäuse bereits eine erste Signalaufbereitung erfolgen, beispielsweise indem ein Signalverstärker und/oder ein analog und/oder digital arbeitender Signalfilter im Sensorgehäuse angeordnet sind. Es wird so eine weniger störanfällige Übertragung des Messsignals an die Auswerteeinheit erzielt.

Weiter kann die Auswerteeinheit einen Speicher zum Abspeichern eines Zeitverlaufs der physiologischen Parameter aufweisen. Die Auswerteeinheit kann zudem eine Überwachungseinrichtung zum Vergleichen der physiologischen Parameter mit vorgegebenen Grenzwerten aufweisen, um in einem Fall, in dem eine Gesundheitsgefahr für die Person erkannt wird, diese oder eine weitere Person gewarnt werden kann. Zu diesem Zweck weist die Auswerteeinheit bevorzugt eine Übertragungseinheit zur Übertragung der physiologischen Parameter auf ein Mobilgerät auf oder ist mit einer in der Steuereinrichtung des elektromotorischen Möbelantriebs vorhandenen Übertragungseinheit gekoppelt. Die Übertragungseinheit ist bevorzugt zur drahtlosen Übertragung der physiologischen Parameter an das Mobilgerät eingerichtet, insbesondere über eine WLAN- oder Bluetooth-Übertragungsstrecke. Wenn die physiologischen Parameter an das Mobilgerät übertragen werden, kann ein Vergleich der physiologischen Parameter mit vorgegebenen Grenzwerten auch im Mobilgerät vorgenommen werden. Zusätzlich kann das Mobilgerät die Handbedienung für den Möbelantrieb darstellen, was Material und Kosten für eine separate Handbedienung einspart und eine komfortablere Bedienung zulässt, da nicht mehrere separate Geräte griffbereit im Bereich des Möbels positioniert werden müssen.

In einer weiteren vorteilhaften Ausgestaltung des elektromotorischen Möbelantriebs ist die Auswerteeinheit für die Signale des Sensors zusätzlich zur Erfassung und Auswertung von Vibrationen eingerichtet, die bei der Betätigung eines oder mehrerer der Verstellantriebe auftreten. Auf diese Weise kann als positiver Nebennutzen der Sensor verwendet werden, um eine Fehlfunktion und/oder eine Überlastung und/oder eine Nicht-Belastung eines oder mehrerer der Verstellantriebe im Betrieb zu ermitteln. Die ermittelten Zustände deuten auf bereits eingetretene oder eventuell bevorstehende technische Probleme der Verstellantriebe hin, oder auf eine Fehlnutzung.

Ein erfindungsgemäßes Schlaf- oder Ruhemöbel, insbesondere Bett, zeichnet sich dadurch aus, dass ein zuvor beschriebener Sensor an dem Schlaf- oder Ruhemöbel, an einem Möbelteil des Schlaf- oder Ruhemöbels, befestigt ist. Erfindungsgemäß ist der Sensor an einem Rückenteil des Schlaf- oder Ruhemöbels befestigt, da derart Vibrationen aus dem Brustraum einer das Schlaf- oder Ruhemöbel benutzenden Person besonders gut erfasst werden können, die Rückschlüsse auf Atmung und Kreislauf der Person zulassen. Eine Positionierung des Sensors in einer unteren Hälfte und insbesondere in einem unteren Drittel des Rückenteils des Schlaf- oder Ruhemöbels ist diesbezüglich besonders geeignet. Um Schwingungsmoden und ggf. Resonanzen des Rückenteils auszunutzen, ist es vorteilhaft, den Sensor in einem Bereich eines Schwingungsbauchs einer Eigenschwingungsmode des Rückenteils anzuordnen. Das Rückenteil, oder allgemeiner das Möbelteil, an dem der Sensor befestigt ist, wird dann Teil des Systems zur Aufnahme der Schwingungen bzw. Vibrationen und dient als Schallleiter und gewissermaßen als "Antenne" für die Schwingungen. Erfindungsgemäß ist es der

Sensor zwischen Befestigungspunkten eines Beschlagelements, das das Rückenteil mit einem der Verstellantriebe koppelt, an dem Rückenteil des Schlaf- oder Ruhemöbels zu befestigen. Ein solches Beschlagelement beeinflusst durch seine Stabilität die Eigenschwingungsmoden des Rückenteils. Es ist z.B. als ein Dreibein mit drei voneinander beabstandeten Auflagepunkten zum Rückenteil ausgebildet. Im Bereich zwischen den Auflagepunkten liegt in der Regel ein Schwingungsbauch mit einer großen Schwingungsamplitude.

Die Erfindung wird nachfolgend anhand von Ausführungsbeispielen mithilfe von Figuren näher erläutert. Es zeigen:
- Fig. 1: ein erstes Ausführungsbeispiel eines Schlafmöbels mit einem elektromotorischen Möbelantrieb und einem Sensor in einer isometrischen Ansicht;
- Fig. 2: ein zweites Ausführungsbeispiel eines Schlafmöbels mit einem elektromotorischen Möbelantrieb und einem Sensor in einem schematischen Blockschaltbild;
- Fig. 3: eine Wiedergabe einer zeitlichen Abhängigkeit von Sensordaten;
- Fig. 4: ein drittes Ausführungsbeispiel eines Schlafmöbels mit elektromotorischem Antrieb und einem Sensor in einer isometrischen Ansicht;
- Fig. 5: das Schlafmöbel gemäß Fig. 4 in einer isometrischen Ansicht von schräg unten;
- Fig. 6: ein viertes Ausführungsbeispiel eines Schlafmöbels mit elektromotorischem Antrieb und einem Sensor in einer isometrischen Ansicht;
- Fig. 7: das Schlafmöbel gemäß Fig. 6 in einer isometrischen Ansicht von schräg unten; und
- Fig. 8, 9: jeweils eine isometrische Darstellung eines Sensors mit Sensorgehäuse aus verschiedenen Blickrichtungen;
- Fig. 10: ein Ausführungsbeispiel eines Stützelements für ein Schlafmöbel mit einem Sensor; und
- Fig. 11, 12: jeweils eine isometrische Ansicht des Sensorgehäuses gemäß den Figuren 8 und 9 mit einem Montageadapter.

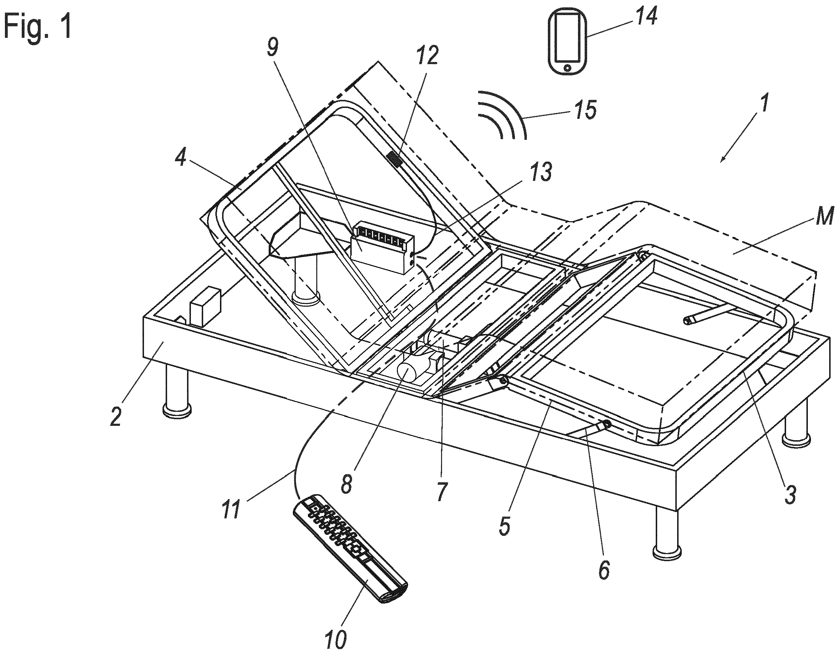

Fig. 1 zeigt ein Bett 1 als Beispiel eines Schlafmöbels mit einem elektromotorischen Möbelantrieb. Das Bett 1 weist wenigstens ein Stützelement 3 zur Aufnahme von z.B. einer Polsterung oder einer Matratze M, auf. Das Bett 1 kann als ein Einzelbett für eine Person oder auch als Doppelbett für mehrere Personen ausgelegt sein. Das Stützelement 3 ist z.B. als ein Lattenrost, als ebene Stützfläche oder dergleichen ausgebildet und an einem Grundelement 2, hier einem Gestell mit Füßen, mit dem das Bett 1 an einem Aufstellort, z.B. Fußboden, aufgestellt ist.

Das Stützelement 3 weist im dargestellten Beispiel ein Rückenteil 4 und ein Beinteil 5 auf, welche relativ zu einem hier festen mittleren Teil oder relativ zu dem Grundelement 2 beweglich gelagert angeordnet sind. Diese bewegliche Anordnung ist hier mittels eines so genannten Bewegungsbeschlags 6 realisiert. Die Bewegung ist verschiebbar und/oder schwenkbar ausgebildet.

Das beweglich gelagerte Rückenteil 4 und das Beinteil 5 sind jeweils mit einem elektromotorischen Verstellantrieb 7, 8 gekoppelt. So ist das Rückenteil 4 mit dem elektromotorischen Verstellantrieb 7 gekoppelt. Zur Bewegung bzw. Verstellung des Beinteils 5 ist der elektromotorische Verstellantrieb 8 vorgesehen.

Die elektromotorischen Verstellantriebe 7, 8 sind vorliegend als Linearantriebe ausgebildet. Die Linearantriebe weisen einen oder eine Anzahl Elektromotoren auf, wobei jedem Motor ein Drehzahlreduziergetriebe mit wenigstens einer Getriebestufe nachgeschaltet ist. Dem Drehzahlreduziergetriebe kann ein weiteres Getriebe, beispielsweise in Form eines Gewindespindelgetriebes, nachgeschaltet sein, welches aus der Drehbewegung des Motors eine Linearbewegung eines Abtriebsgliedes erzeugt. Das letzte Getriebeglied oder ein damit verbundenes weiteres Glied bildet das Abtriebsglied. Das Abtriebsglied des jeweiligen elektromotorischen Verstellantriebs steht mit dem jeweiligen Möbelbauteil (Rückenteil 4, Beinteil 5) oder alternativ mit einem mit dem Grundelement 2 verbundenes Bauteil in Verbindung, so dass bei einem Betrieb des Elektromotors des jeweiligen Verstellantriebs 7, 8 die beweglichen Möbelbauteile 4, 5 relativ zueinander bzw. relativ zum Grundelement 2 verstellt werden.

Die elektromotorischen Verstellantriebe 7, 8 sind mit einer Steuereinrichtung 9 verbunden. Diese Verbindung kann z.B. als steckbare Kabelverbindung ausgeführt sein, was hier nicht näher dargestellt ist. Die Steuereinrichtung 9 weist eine elektrische Versorgungseinheit auf, welche die elektrische Energie, z.B. aus einem Stromversorgungsnetz, für die elektromotorischen Verstellantriebe 7, 8 bereitstellt. Dazu ist die Steuereinrichtung 9 über ein in diesem Beispiel nicht gezeigtes Netzkabel mit einem Netzstecker mit einem Netzanschluss verbindbar. Der Netzstecker leitet über das Netzkabel die eingangsseitige Netzspannung zu der elektrischen Versorgungseinheit der Steuereinrichtung 9, welche sekundärseitig eine Kleinspannung in Form einer Gleichspannung abgibt.

Alternativ hierzu ist der Steuereinrichtung 9 eine externe netzabhängige Spannungsversorgung mit Netzeingang und mit sekundärseitigem Kleinspannungsausgang vorgeschaltet, welche über die Leitung die Kleinspannung in Form einer Gleichspannung zuführt.

In einer alternativen Ausgestaltung ist die Steuereinrichtung nicht in einem separaten Gehäuse angeordnet, sondern in einen der Verstellantriebe 7, 8 integriert. Dieser Verstellantrieb stellt dann einen Hauptantrieb dar, an den ggf. weitere Verstellantriebe angeschlossen werden können.

In einer weiteren alternativen Ausgestaltung eines elektromotorischen Möbelantriebs kann die Steuereinrichtung verteilt im System angeordnet sein, derart, dass jeder der Verstellantriebe 7, 8 selbst über eine Motorsteuerung verfügt und eine Bus-Kommunikationsschnittstelle aufweist, über die die Verstellantriebe 7, 8 untereinander und mit weiteren Komponenten verbunden sind. Dabei kann vorgesehen sein, dass wenigsten einer der Verstellantriebe 7, 8 ein eigenes Netzteil zu seiner Stromversorgung oder zur Versorgung mehrerer oder aller vorhandenen Verstellantriebe und/oder ggf. weiterer Systemkomponenten aufweist.

Es ist eine Handbedienung 10 vorgesehen, die Bedienelemente aufweist, mit denen die elektromechanischen Verstellantriebe 7, 8 über die Steuereinrichtung 9 steuerbar sind. Die Handbedienung 10 kann in einem Ausführungsbeispiel über ein Kabel mit der Steuereinrichtung 9 verbunden sein. Alternativ kann die Handbedienung 10 mit einer Übertragungseinrichtung für eine drahtlose Übertragung von Signalen zur Steuereinrichtung 9 versehen sein. Die drahtlose Übertragung kann durch eine Funkübertragungsstrecke, eine optische Übertragungsstrecke (z.B. für Infrarotlicht) und/oder eine Ultraschallübertragungsstrecke realisiert sein, wobei die Steuereinrichtung 9 mit einer jeweiligen entsprechenden Empfangseinheit ausgerüstet ist. Weiter alternativ kann die Handbedienung auch die Steuereinrichtung für die Verstellantriebe bilden, z.B. indem der Betriebsstrom der Verstellantriebe direkt über Schalter der Handbedienung geschaltet wird.

Die Bedienelemente können beispielsweise als Taster und/oder Schalter ausgebildet sein. Ferner kann die Handbedienung 10 mit einem Meldeelement, z.B. einer Leuchtdiode oder einer Anzeigeeinheit, ausgerüstet. Das Meldeelement dient z.B. zur Funktionsanzeige bzw. Rückmeldung, Fehleranzeige usw..

Anmeldungsgemäß ist bei dem dargestellten Bett 1 ein Sensor 12 vorgesehen, der Vibrationen und/oder Schall detektiert. Der Sensor 12 ist im dargestellten Ausführungsbeispiel an einem Rahmenbauteil des Rückenteils 4 befestigt. Die Befestigung kann eine Schraub- oder Niet-oder Klebeverbindung sein oder auch eine Rast- oder Klemmverbindung, beispielsweise mithilfe einer Federklemme, die das entsprechende Rahmenbauteil umgreift. Der Sensor 12 ist als piezoelektrisches Bauteil ausgebildet und weist einen derartig arbeitenden Schall- bzw. Schwingungsaufnehmer auf und ist empfindlich für Schwingungen der Unterlage, auf der er befestigt ist, vorliegend also für Schwingungen (Vibrationen), die der Rahmen des Rückenteils 4 erfährt. Derartige Vibrationen umfassen auch Körperschall, der von dem Rückenteil weitergeleitet wird. Zusätzlich kann der Sensor 12 empfindlich für (Luft-) Schallwellen sein und in diesem Sinne als ein Mikrofon fungieren. Ein weiterer geeigneter Sensor ist ein elektromechanischer Sensor, z.B. ein mikromechanischer Beschleunigungssensor.

Der Sensor 12 ist über ein Sensorkabel 13 mit der Steuereinrichtung 9 verbunden. Falls benötigt, wird über das Sensorkabel 13 eine Stromversorgung für den Sensor 12 bereitgestellt und es werden vom Sensor 12 ausgegebene Signale an die Steuereinrichtung 9 weitergeleitet. In einer alternativen Ausgestaltung kann der Sensor 12 über eine drahtlose Verbindung, beispielsweise eine Funkverbindung, mit der Steuereinrichtung 9 gekoppelt sein. In dem Fall ist der Sensor 12 mit einer eigenen Energieversorgung, beispielsweise in Form einer gegebenenfalls wiederaufladbaren Batterie versehen.

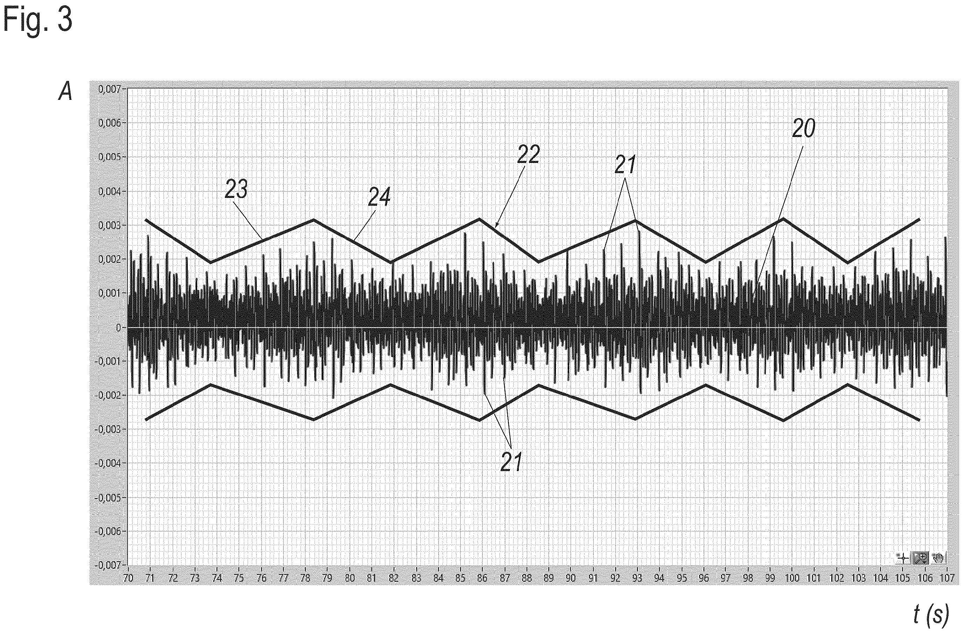

Die Steuereinrichtung 9 umfasst eine Auswerteeinheit zur Verarbeitung und Auswertung der vom Sensor 12 gelieferten Signale. Die Auswerteeinheit umfasst z.B. Verstärker und Filtereinheiten, die es ermöglichen, aus dem vom Sensor 12 übermittelten Signal auf bestimmte Körperfunktionen einer sich im Bett 1 befindenden Person zu schließen. Insbesondere ist die Auswerteeinheit dazu eingerichtet, aus den Signalen des Sensors 12 physiologische Parameter der Person zu ermitteln. Solche Parameter betreffen beispielsweise Herz- und Kreislauffunktionen und umfassen z.B. eine Herzfrequenz und eine Atemfrequenz. Weiter kann ermittelt werden, ob die sich im Bett befindende Person schnarcht. Zudem werden Bewegungen der Person erfasst. Details zur Ermittlung der genannten Parameter aus den Signalen des Sensors 12 werden weiter unten im Zusammenhang mit Fig. 3 näher erläutert.

Die bestimmten Parameter werden entweder unmittelbar oder nach Zwischenspeicherung in der Steuereinrichtung 9 als drahtlose Signale 15 an ein Mobilgerät 14 übertragen. Das Mobilgerät 14 kann insbesondere ein handelsübliches Mobiltelefon ("Smartphone") oder ein Tablet-Computer sein und ist mit einer entsprechenden Software ("App") ausgestattet, die eine Auswertung und bevorzugt grafische Darstellung der Zeitabhängigkeit der ermittelten Schlafparameter ermöglicht. Als Übertragungsweg für die drahtlosen Signale 15 kann beispielsweise WLAN (Wireless Lokal Area Network) oder Bluetooth eingesetzt werden.

Zudem kann in der Steuereinrichtung 9 ein Vergleich der gemessenen physiologischen Parameter mit vorgegebenen Grenzwerten für diese Parameter vorgesehen sein. Werden die ermittelten Parameter unmittelbar, das heißt ohne längere Zwischenspeicherung in der Steuereinrichtung 9, während der Schlafphase an das Mobilgerät 14 übertragen, kann alternativ oder zusätzlich dort ein solcher Vergleich erfolgen. Bei Über- oder Unterschreiten der Grenzwerte bzw. bei einem Verlassen eines oder mehrerer der Parameter aus einem vorgegebenen Bereich ist vorgesehen, dass die Steuereinrichtung 9 bzw. das Mobilgerät 14 ein Alarmsignal ausgibt. Dieses Alarmsignal kann optisch und/oder akustisch unmittelbar von der Steuereinrichtung 9 bzw. dem Mobilgerät 14 ausgegeben werden. Alternativ oder zusätzlich kann vorgesehen sein, dass das Mobilgerät 14 eine Alarmmeldung über eine weitere, hier nicht dargestellte drahtlose Übertragungsstrecke (z.B. WLAN, Mobilfunknetz) abgibt. Auf diese Weise kann eine weitere Person benachrichtigt werden, falls sich ungewöhnliche Schlafparameter zeigen. Das dargestellte Bett 1 bzw. der elektromotorische Möbelantrieb mit dem Sensor 12 kann so auch zur klinischen Überwachung bzw. zur Patientenüberwachung oder zur Überwachung von Kleinkindern zum Schutz vor plötzlichem Kindstod eingesetzt werden. Beispielsweise kann eine Alarmmeldung abgegeben werden, wenn eine Person das Bett verlassen hat, ggf. wenn eine Person das Bett seit einer vorbestimmten Zeit verlassen hat oder wenn keine oder nur als kritisch angesehene physiologische Parameter detektiert werden.

Bei dem dargestellten Ausführungsbeispiel ist der Sensor 12 an einem Rahmenelement des Rückenteils 4 angeordnet. Andere Anordnungen am Schlafmöbel, also dem dargestellten Bett 1, oder mit ihm verbundener Elemente, wie der aufgelegten Matratze M sind möglich. Der Sensor 12 kann beispielsweise an einem hier nicht sichtbaren Lattenrost, das auf das Stützelement 3 aufgelegt ist, montiert sein. Auch eine gegen Verrutschen sichere Montage in oder an der Matratze M ist möglich. Weiter ist die Verwendung mehrerer Sensoren 12, die an unterschiedlichen Stellen im oder am Bett 1 positioniert sind, möglich.

Die Verbindung mit der Steuereinrichtung 9, die innerhalb des Betts 1 angeordnet ist, verhindert, dass das Sensorkabel 13 außerhalb des Bettes 1 verlegt werden muss. Die Fixierung des Sensors 12 im oder am Bett 1 stellt eine jederzeit korrekte Positionierung des Sensors 12 und damit eine zuverlässige Auswertung der Daten des Sensors 12 sicher.

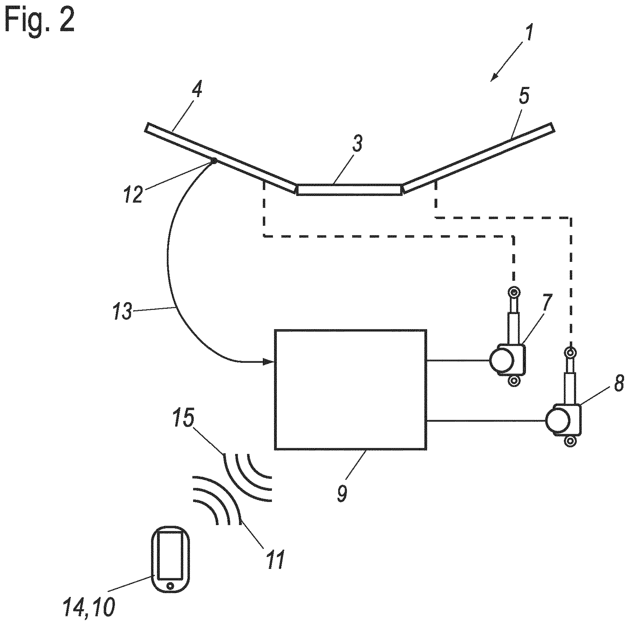

Fig. 2 zeigt ein zweites Ausführungsbeispiel eines Schlafmöbels mit elektromechanischem Möbelantrieb und einem integriertem Sensor 12 in einem schematischen Blockschaltbild. Wiederum ist ein Bett 1 als Beispiel eines Schlafmöbels dargestellt. Gleiche Bezugszeichen kennzeichnen beim Ausführungsbeispiel der Fig. 2 gleiche oder gleich wirkende Elemente wie bei Fig. 1.

In seinem Grundaufbau entspricht der elektromotorische Möbelantrieb gemäß Fig. 2 dem in Fig. 1 gezeigten. Auf die vorstehende Beschreibung wird hiermit verwiesen.

Im Unterschied zum Ausführungsbeispiel der Fig. 1 übernimmt vorliegend das Mobilgerät 14 auch die Funktion der Handbedienung 10. Wiederum ist eine entsprechende Software ("App") für die Funktion als Handbedienung 10 auf dem Mobilgerät installiert.

Die Übertragungsstrecke zwischen dem Mobilgerät 14 und der Steuereinrichtung 9 ist bidirektional ausgeführt, so dass Steueranweisungen an die Verstellantriebe 7, 8 von dem als Handbedienung genutzten Mobilgerät 14 an die Steuereinrichtung 9 gesendet werden können, und Daten, die den Schlafzustand betreffen, von der Steuereinrichtung 9 an das Mobilgerät 14 übertragen werden können.

Bei den Ausführungsbeispielen der Figuren 1 und 2 ist die Auswerteeinheit für die Signale des Sensors 12 in die Steuereinrichtung 9 integriert. Alternativ ist es möglich, die Auswerteeinheit separat von der Steuereinrichtung 9 in einem eigenen Gehäuse auszubilden. Zur Übertragung der ermittelten physiologischen Parameter kann die Auswerteeinheit dann mit der Steuereinrichtung 9 elektrisch gekoppelt sein. Auch ist eine Nutzung einer solchen autarken Auswerteeinheit losgelöst von der Steuereinrichtung 9 möglich, insbesondere wenn im Gehäuse der Auswerteeinrichtung bereits eine Übertragungseinheit zur drahtlosen Übertragung der ermittelten physiologischen Parameter und/oder vorverarbeiteter Signale des Sensors 12 an das Mobilgerät 14 vorhanden ist.

Fig. 3 zeigt einen Ausschnitt eines gemessenen Signals 20 des Sensors 12 in einem Diagramm. Auf der horizontalen Achse ist der Zeitverlauf t in Sekunden angegeben. Auf der vertikalen Achse ist eine Signalamplitude A in willkürlichen Einheiten dargestellt.

Der gezeigte Ausschnitt des Signalverlaufs des Signals 20 ist während einer ruhigen Schlafphase ohne Bewegung und ohne Schnarchen der beobachteten Person. Eine Bewegung der Person äußert sich in Amplituden, die die dargestellte um einen Faktor von einigen 10-100 übersteigen. Bewegungen lassen sich daher sehr leicht identifizieren. Auch ein Schnarchen und die damit einhergehenden Vibrationen sind von dem dargestellten Signalverlauf deutlich unterscheidbar, da sie sich in einer um ein mehrfaches größeren Amplitude widerspiegeln.

In dem in Fig. 3 gezeigten Verlauf des Signals 20 sind regelmäßige Peaks 21 beobachtbar, die vom Herzschlag der Person herrühren und nachfolgend Herzschlag-Peaks 21 genannt werden. Aus dem Abstand der Herzschlag-Peaks 21 kann eine Herzfrequenz ermittelt werden. Der zeitliche Abstand benachbarter Herzschlag-Peaks 21 lässt Aussagen über die Puls-Gleichmäßigkeit zu, die ein Maß für die Tiefe des Schlafes sein kann.

Weiterhin ist in Fig. 3 zu erkennen, dass die Amplitude der Herzschlag-Peaks 21 regelmäßig mit einer geringeren Frequenz variiert. Diese Variation wird durch eine Einhüllende 22 wiedergegeben. Die Einhüllende 22 zeigt sich abwechselnde ansteigende Flanken 23 und abfallende Flanken 24. Der Verlauf der Einhüllenden 22 ist mit der Atmung der Person korreliert. Die ansteigenden Flanken 23 kennzeichnen eine Einatemphase und die absteigende Flanke 24 eine Ausatemphase.

Das Beispiel der Fig. 3 zeigt, wie aus den Signalen des Sensors 12 auf Herz-Kreislaufparameter geschlossen werden kann, vorliegend auf Puls- und Atmung. Auf ähnliche Weise können weitere Schlafparameter, wie Bewegungszustände und ein Schnarchen ermittelt werden.

Zur Auswertung der Signale 20 erfolgt eine Filterung der Rohsignale des Sensors 12, insbesondere mittels eines Tiefpassfilters. Auch die Verwendung eines Bandpassfilters mit geeigneten Eckfrequenzen ist möglich. Tiefpass-bzw. Bandpassfilter dienen der Eliminierung von Störfrequenzen. Bevorzugt erfolgt die Verarbeitung der Signale mit Hilfe eines digitalen Signalprozessors (DSP).

Der Sensor 12 kann zusätzlich oder alternativ auch einer Überwachung der korrekten Funktion des elektromotorischen Antriebs dienen. Eine Betätigung der Verstellantriebe 7, 8 führt zu einer Bewegung der bewegten Möbelteile, beispielsweise des Rückenteils 4 und/oder des Beinteils 5. Zusätzlich resultiert die Betätigung der Verstellantriebe 7, 8 in Vibrationen dieser Möbelteile und auch des gesamten Möbels, die ebenfalls vom Sensor 12 erfasst werden. Diese Vibrationen treten in einem typischen Frequenzbereich auf. Der Signalverlauf spiegelt die Motorbewegung der Verstellantriebe 7, 8 wider. Ein erster typischer relevanter Frequenzbereich liegt im Bereich der Motordrehzahl der Motoren der Verstellantriebe 7, 8. In diesem Frequenzbereich zeigen sich Fehler am Motor selbst oder einem Abtriebszahnrad. Ein weiterer typischer relevanter Frequenzbereich entspricht einem ganzzahligen Bruchteil gemäß einem Übersetzungsverhältnis des Getriebes, das etwa 1:30 bis 1:50 beträgt. In diesem Frequenzbereich deuten sich Fehler in nachgeordneten Getriebestufen oder Wälzlagern an. Ein dritter typischer Frequenzbereich liegt im Bereich von Quietschgeräuschen von Scharnieren, die Teil eines Möbelbeschlags sind. Form und Amplitude sind zum einen typisch für den verwendeten Verstellantrieb 7, 8, zum anderen geben sie Aufschluss über die korrekte Funktion der Verstellantriebe 7, 8 sowie deren Verschleißzustand.

Auch eine Überbelastung einer der Verstellantriebe 7, 8 kann anhand der Signalform der Signale des Sensors 12 erkannt werden. Der Sensor 12 kann somit beispielsweise als Einklemmschutz fungieren, wobei die Steuereinrichtung 9 bei Überbelastung eines der Verstellantriebe 7, 8 diesen Antrieb stoppt bzw. in Gegenrichtung laufen lässt. Auch eine Unterlast am Verstellantrieb 7, 8 kann ein Indiz für ein Einklemmen sein, beispielsweise wenn ein Möbelteil (Rückenteil 4, Beinteil 5) abgelassen wird und der Verstellantrieb 7, 8 nahezu kraftlos betrieben wird, deutet dieses auf ein Einklemmen eines Körperteils unter dem sich hinab senkenden bewegten Möbelteil hin. Ein belastungslos betriebener Verstellantrieb 7, 8 ist ebenfalls anhand der Signale des Sensors 12 identifizierbar.

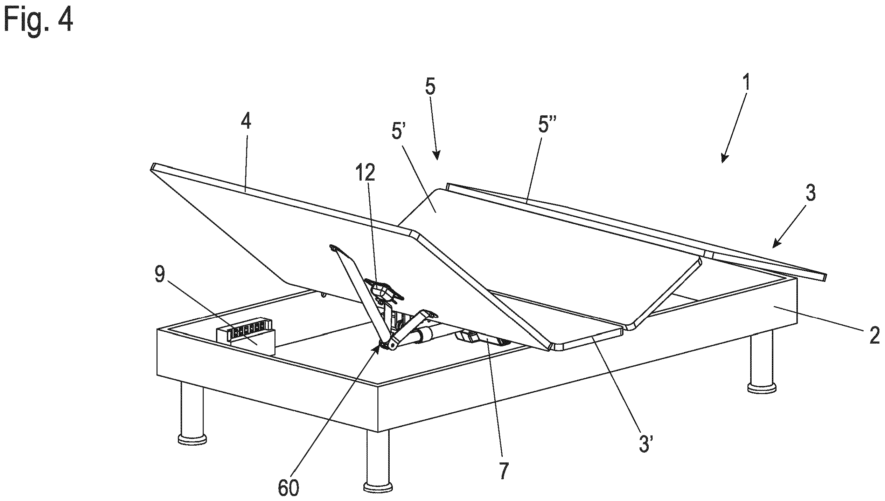

In den Figuren 4 bis 7 sind nachfolgend zwei weitere Ausführungsbeispiele eines Betts 1 als Beispiel eines Schlafmöbels mit einem elektromotorischen Möbelantrieb mit Verstellantrieben 7, 8 und einer Steuereinrichtung 9 sowie einem Sensor 12 zur Erfassung von Vibrationen und/oder Schall dargestellt. In allen Ausführungsbeispielen kennzeichnen gleiche Bezugszeichen gleiche bzw. gleichwirkende Elemente wie bei den zuvor beschriebenen Beispielen.

Aus Gründen der Übersichtlichkeit sind bei den Figuren 4 bis 7 eine Handbedienung 10 mit Übertragungsstrecke 11 sowie ein Mobilgerät 14 und die mit diesem ausgetauschten drahtlosen Signale 15, wie sie in den Figuren 1 und 2 gezeigt sind, nicht dargestellt. Es versteht sich, dass die Steuereinrichtung 9 in diesen Ausführungsbeispielen auf gleiche oder vergleichbare Art und Weise mit den genannten Handbedienungen 10 bzw. Mobilgeräten 14 gekoppelt sein können, wie es in den Figuren 1 und 2 dargestellt ist und wie im Zusammenhang mit diesen Figuren beschrieben wird.

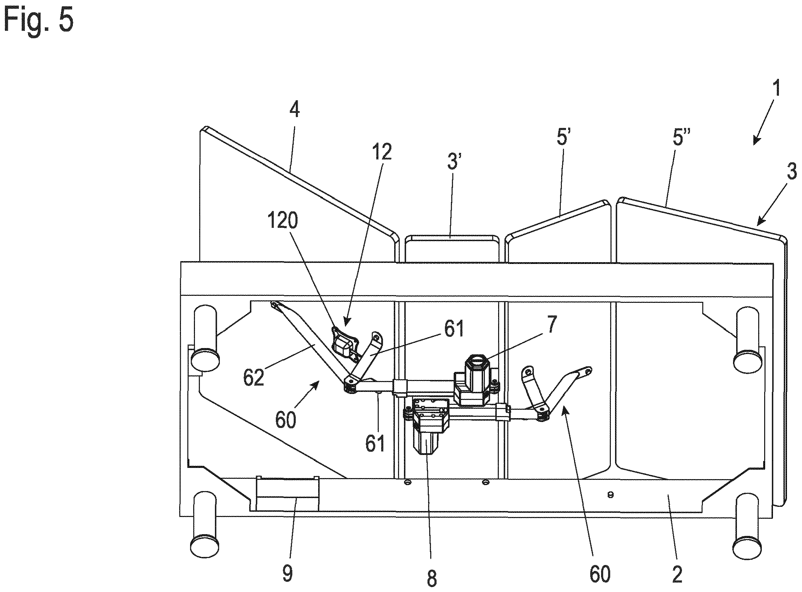

In den Figuren 4 und 5 ist das dritte Ausführungsbeispiel des Betts 1 in jeweils einer isometrischen Ansicht aus zwei verschiedenen Blickrichtungen wiedergegeben. Wiederum weist das Bett 1 ein Gestell mit Füßen auf, das ein Grundelement 2 darstellt. Es versteht sich, dass anstelle des rahmenartigen Gestells mit den separat ausgebildeten Füßen auch ein eher kastenförmiges Gestell mit vier Seitenwänden, die bis zum Boden reichen, ausgebildet sein kann.

Das Grundelement 2 trägt ein Stützelement 3, das eine Polsterung, insbesondere eine hier nicht dargestellte Matratze aufnimmt. Auch wenn das wiedergegebene Bett 1 ein Einzelbett für eine Person ist, kann es in gleicher Weise als ein Doppelbett für mehrere Personen ausgelegt sein.

Vorliegend weist das Stützelement 3 keinen tragenden Rahmen auf, der z. B. ein Lattenrost als Unterlage der Polsterung bzw. der Matratze aufnimmt, sondern im Wesentlichen selbsttragende Platten. Ein derartiges Bettkonzept wird beispielsweise bei sogenannten Boxspringbetten umgesetzt, bei denen die Matratze dicker als herkömmliche Matratzen ist und Federelemente beinhaltet, die ohne eine federnde Unterlage, wie sie beispielsweise ein Lattenrost darstellt, einen ausreichenden Schlafkomfort bietet.

Das Stützelement 3 weist wiederum mehrere gegeneinander verstellbare Abschnitte auf, konkret einen mittleren, nicht schwenkbaren Abschnitt 3', ein diesem gegenüber schwenkbares Rückenteil 4 und ein zweiteiliges Beinteil 5. Ein erster Abschnitt 5' des Beinteils 5 ist dabei schwenkbar gegenüber des Stützelements 3 gekoppelt. Ein zweiter Abschnitt 5" des Beinteils 5 ist an einer freien Querseite des ersten Abschnitts 5' gelenkig mit diesem verbunden und wird entsprechend an der Verbindungslinie der beiden Abschnitte 5', 5" mit angehoben. Entlang der Verbindungslinien zwischen dem nicht schwenkbaren Abschnitt 3' des Stützelements 3 und dem Rückenteilteil 4 bzw. dem ersten Abschnitt 5' des Beinteils 5, sowie an der Verbindungslinie zwischen dem ersten und dem zweiten Abschnitt 5', 5" des Beinteils 5 sind Schwenkbeschläge, z. B. in Form von Scharnierleisten angeordnet. Der nicht schwenkbaren Abschnitt 3' ist vorliegend ortsfest zu dem Grundelement 2.

Fig. 5 zeigt das Bett 1 des dritten Ausführungsbeispiels in einer Ansicht von schräg unten. In dieser Ansicht sind der elektromotorische Möbelantrieb und seine Kopplung mit den verschiedenen Teilen des Stützelements 3 gut zu erkennen. Wie bei dem ersten und zweitem Ausführungsbeispielen sind zwei Verstellantriebe 7, 8 vorgesehen, die beide an jeweils einer ihrer Seiten mit dem nicht schwenkbaren Abschnitt 3' des Stützelements 3 gekoppelt sind. Mit einem bewegbaren, hier linear ausfahrbaren Abtriebseite sind die beiden Verstellantriebe 7, 8 jeweils mit einem Beschlagelement 60 gekoppelt, das an dem Rückenteil 4 bzw. dem ersten Beinteil 5' montiert ist. Das Beschlagelement hat hier jeweils die Form eines Dreibeins, wobei zwei Querstützen 61 den Krafteinleitungspunkt des jeweiligen Verstellantriebs 7, 8 seitlich festlegen und eine Längsstütze 62 die Verstellkraft in das Rückenteil 4 bzw. das Beinteil 5 einleitet.

Wie in den Figuren 4 und 5 ersichtlich ist, ist der Sensor 12 mit einem Sensorgehäuse 120 auf der Unterseite des Rückenteils 4 montiert. Die Montageposition liegt vorteilhafterweise in etwa mittig in der Querrichtung des Bettes 1 und in etwa im Bereich des unteren, dem nicht schwenkbaren Abschnitt 3' des Stützelements 3 benachbarten Drittels des Rückenteils 4 im Hinblick auf die Längsposition innerhalb des Bettes 1.

Besonders vorteilhaft ist der Sensor 12 innerhalb des Dreiecks montiert, dessen Eckpunkte durch die Befestigungsstellen des Beschlagelements 60 am Rückenteil 4 definiert ist. In der dargestellten Position befindet sich der Sensor 12 in einer physiologisch sinnvollen Position, da sich der Sensor 12 in etwa auf Höhe des Brustkorbs befindet, von dem aus im Schlaf die Herzschlagsbewegung und auch Atembewegungen und Atemgeräusche über die Matratze auf das Rückenteil 4 des Betts 1 übertragen werden. Weiterhin sind für eine Signalerfassung durch den Sensor 12 Eigenschwingungen des Rückenteils 4 relevant. Diese Eigenschwingungen werden durch die Form und Größe des Rückenteils 4 selbst bestimmt und auch durch die Befestigungsstellen des Beschlagelements 60, das mit dem Verstellantrieb 7 gekoppelt ist.

Bei einer Schwingungsanregung des Rückenteils 4 stellen die Verbindungspunkte zum Beschlagelement 60 Bereiche mit geringer Schwingungsamplitude dar. In einem Abschnitt zwischen diesen Punkten liegt jedoch ein zumindest lokales Maximum der Schwingungsamplitude für eine Schwingungsmode des Rückenteils 4 vor.

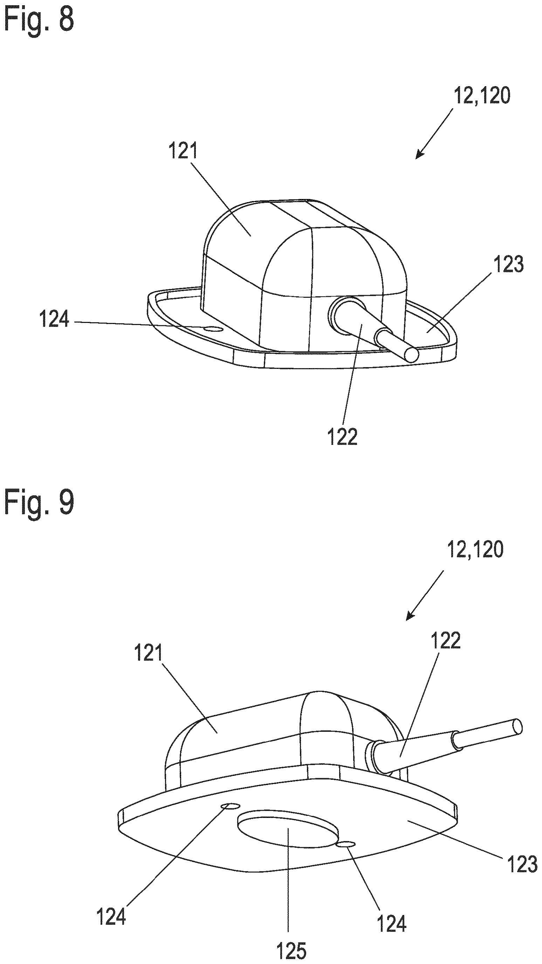

Das in den Figuren 4 und 5 dargestellte Gehäuse 120 des Sensors 12 wird im Zusammenhang mit den Figuren 8 und 9 noch detaillierter erläutert.

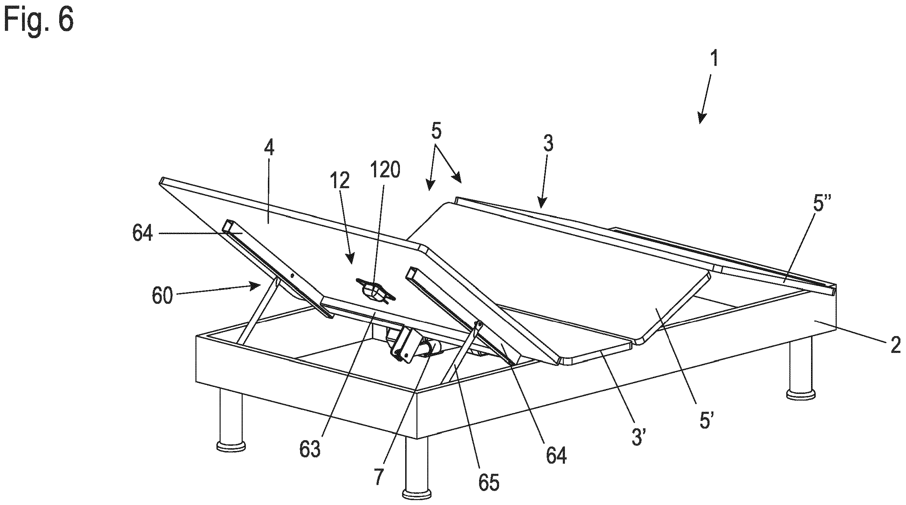

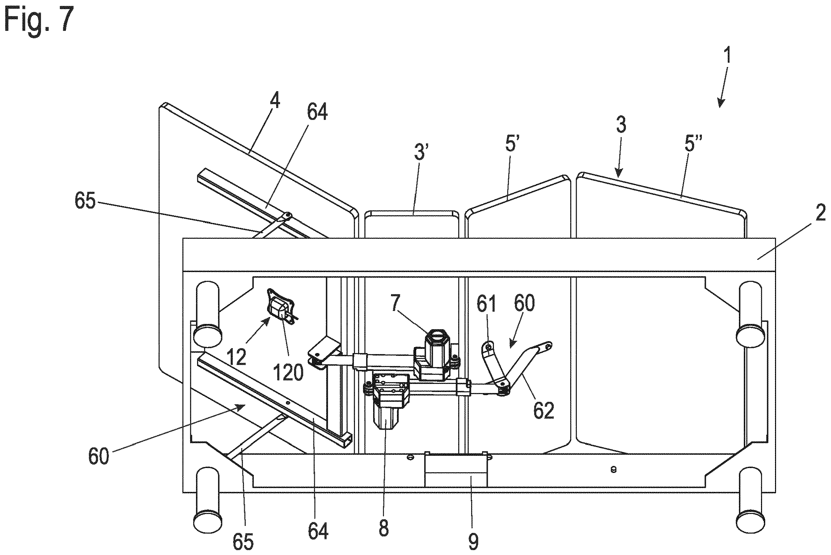

In den Figuren 6 und 7 ist ein weiteres Ausführungsbeispiel eines Betts 1 mit elektromotorischem Antrieb und Sensor 12 zur Aufnahme von Vibration und/oder Schall dargestellt. Fig. 6 zeigt eine isometrische Ansicht von schräg oben und Fig. 7 eine Ansicht mit Blickrichtung auf die Unterseite des Betts 1.

Wie beim Ausführungsbeispiel der Figuren 4 und 5 weist das Bett 1 auch hier ein Stützelement 3 mit plattenförmigen Abschnitten auf: einen nicht schwenkbaren Abschnitt 3', ein diesem gegenüber schwenkbares Rückenteil 4 und ein Beinteile 5, von dem eine erster Abschnitt 5'elektromotorisch gegenüber dem nicht schwenkbaren Abschnitt 3' verschwenkbar ist.

Unterschiedlich zu dem Ausführungsbeispiel der Figuren 4 und 5 sind bei diesem Ausführungsbeispiel die Art der Kraftübertragung und die Bewegungskinematik des Rückenteils 4. Am Rückenteil 4 ist zur Kraftübertragung ein u-förmiger Rahmen als Beschlagelement 60 angeschraubt, dessen Basis 63 in Querrichtung des Betts 1 im Bereich der Verbindung von Beinteil 4 und nicht schwenkbaren Abschnitt 3' verläuft. Zwei von dieser Basis 63 ausgehende Schenkel 64 verlaufen in Längsrichtung des Betts 1 jeweils im Seitenbereich des Rückenteils 4. Sie sind durch Hebel 65 am Grundelement 2 des Betts 1 angelenkt. Bei dieser Ausführungsform des Betts 1 ist der nicht schwenkbare Abschnitt 3' in Längsrichtung verschiebbar am Grundelement 2 gelagert. Bei einem Hochschwenken des Rückenteils 4 behält dieses im Wesentlichen seine Längsposition bei und der nicht schwenkbare Abschnitt 3' und das Beinteil 5 folgen der Bewegung der unteren Verbindungslinie zwischen dem Rückenteil 4 und dem nicht schwenkbare Abschnitt 3'.

Auch hierbei ist das Sensorelement 12 in Querrichtung des Betts 1 mittig und in Längsrichtung des Betts 1 im unteren Drittel des Rückenteils 4 angeordnet. Ebenso wie beim Ausführungsbeispiel der Figuren 4 und 5 ist der Sensor in einem Bereich des Rückenteils 4 montiert, der innerhalb der Unterstützungspunkte durch den Möbelbeschlag, mit dem das Rückenteil 4 mit dem Verstellantrieb 7 gekoppelt ist, liegt.

In den Figuren 8 und 9 ist jeweils eine isometrische Darstellung des Sensors 12 mit dem Sensorgehäuse 120 wiedergegeben, das bereits im Zusammenhang mit den Figuren 4 bis 7 dargestellt ist. Das Sensorgehäuse 120 ist in Fig. 8 in einer Schrägansicht auf seine Oberseite und in Fig. 9 in einer Schrägansicht mit Blick auf seine Unterseite wiedergegeben.

Der Sensor 12 dient der Aufnahme von Schwingungen, insbesondere Körperschallschwingungen, die von der Komponente des Betts, an der er montiert ist, auf den Sensor 12 übertragen werden. Wie bereits im Zusammenhang mit den Figuren 1 und 2 erwähnt ist, kann der Sensor 12 verschiedene physikalische Grundprinzipien zur Umsetzung von Schall in ein elektrisches Signal nutzen, beispielsweise ein elektromagnetisches Prinzip, bei dem eine Spule und ein Permanentmagnet sich relativ zueinander bewegen. Ebenfalls ist ein kapazitives Prinzip möglich. Weiter kann der Sensor 12 sich des piezoelektrischen Effekts bedienen, indem piezoelektrisch aktives Material zwischen zwei plattenförmigen metallischen Elektroden angeordnet wird. Ein derartiger Sensor ist insbesondere für eine Biegeschwingung sensitiv. Ein weiterer geeigneter Sensor ist ein elektromechanischer Sensor, z.B. ein mikromechanischer Beschleunigungssensor.

Das dargestellte Sensorgehäuse 120 weist ein kuppelförmiges Gehäuseteil 121 auf, in dem der eigentliche Schall- bzw. Schwingungsaufnehmer angeordnet ist. Eine Kabeldurchführung 122, vorzugsweise mit Knickschutz, dient der Durchführung des Sensorkabels 13. Das kuppelförmige Gehäuseteil 121 ist mittig auf einer Montageplatte 123 angeordnet. Diese weist Befestigungslöcher 124 auf, beispielsweise für eine Schraubmontage.

Wie in Fig. 9 ersichtlich ist, ist an der Unterseite der Montageplatte 123, die dem Möbelteil, beispielsweise dem Rückenteil 4 zugewandt ist, eine Erhöhung 125 angeordnet, mit der der Sensor 12 bei Montage auf einer ebenen Fläche an dieser Fläche anliegt. Eine Schwingungsübertragung erfolgt somit insbesondere im Bereich der Erhöhung 125. Im Inneren des Gehäuses 120 ist der eigentliche Schall- bzw. Schwingungsaufnehmer mit seinem schwingungsempfindlichen Bereich bevorzugt über der Erhöhung 125 angeordnet. Gegebenenfalls kann dazu die Erhöhung 125 nach innen hin fortgesetzt sein, sodass eine unmittelbare Übertragung auf den schwingungsempfindlichen Abschnitt des Schall- bzw. Schwingungsaufnehmer erfolgt. Auf diese Weise wird Körperschall möglichst unmittelbar auf den Schall- bzw. Schwingungsaufnehmer übertragen.

Im kuppelförmigen Gehäuseteil 121 ist zudem genügend Bauraum für eine Auswerteelektronik vorhanden, sodass eine Signalaufarbeitung des Sensorsignals unmittelbar im Sensorgehäuse 120 erfolgen kann. Diese Signalaufarbeitung kann eine Verstärkung und/oder Filterung des vom Schall- bzw. Schwingungsaufnehmer abgegebenen Signals beinhalten. Im Sensorkabel 13 können entsprechende Stromversorgungsleitungen für die Auswerteelektronik im Sensorgehäuse 120 vorgesehen sein.

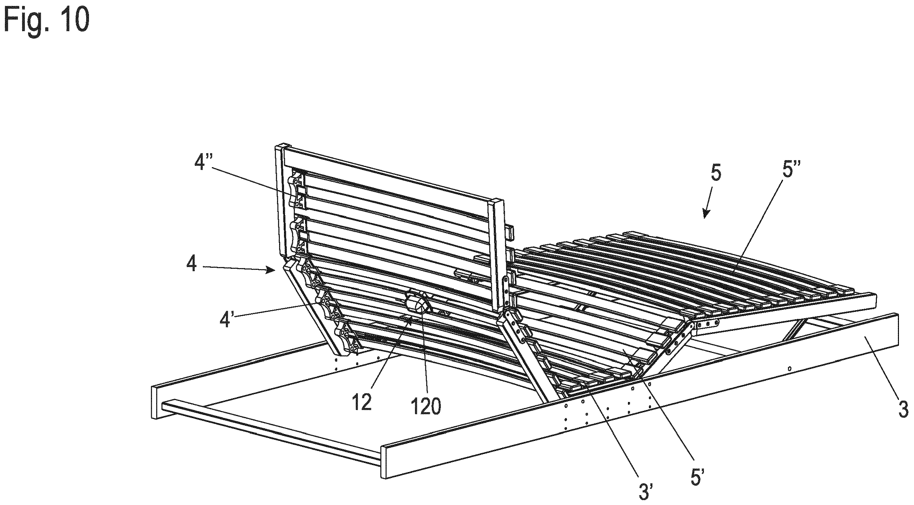

In Fig. 10 ist ein weiteres Ausführungsbeispiel eines Stützelements 3, das in einem hier nicht dargestellten Bett eingesetzt werden kann, wiedergegeben. Anders als die in den Figuren 4-7 dargestellten Stützelemente 3 mit plattenförmigen Abschnitten, weist das in Fig. 10 dargestellte Stützelement 3 eine Rahmenkonstruktion auf, die einzelne Latten trägt. Ein solcher Lattenrost kann in Verbindung mit dünneren Matratzen als bei den zuvor genannten Boxspringbetten eingesetzt werden, da dar Lattenrost selbst eine gewisse Federwirkung beisteuert. Aus Gründen der Übersichtlichkeit sind Verstellantriebe des elektromotorischen Antriebs hier nicht eingezeichnet. Das Stützelement 3 umfasst wiederum ein Rückenteil 4, das vorliegend zwei zueinander schwenkbare Abschnitte 4', 4" aufweist, einen nicht schwenkbaren Abschnitt 3' und ein Beinteil 5 mit den Abschnitten 5' und 5".

Am Stützelement 3 ist am Rückenteil 4 wiederum ein Sensor 12 angeordnet, um Körperschall und auch luftübertragenden Schall zur weiteren Auswertung aufzunehmen. Der Sensor 12 ist bevorzugt wiederum mittig in Querrichtung des Stützelements 3 und damit des Betts angeordnet. In Längsrichtung ist der Sensor 12 im unteren bis mittleren Bereich des Rückenteils 4 angeordnet.

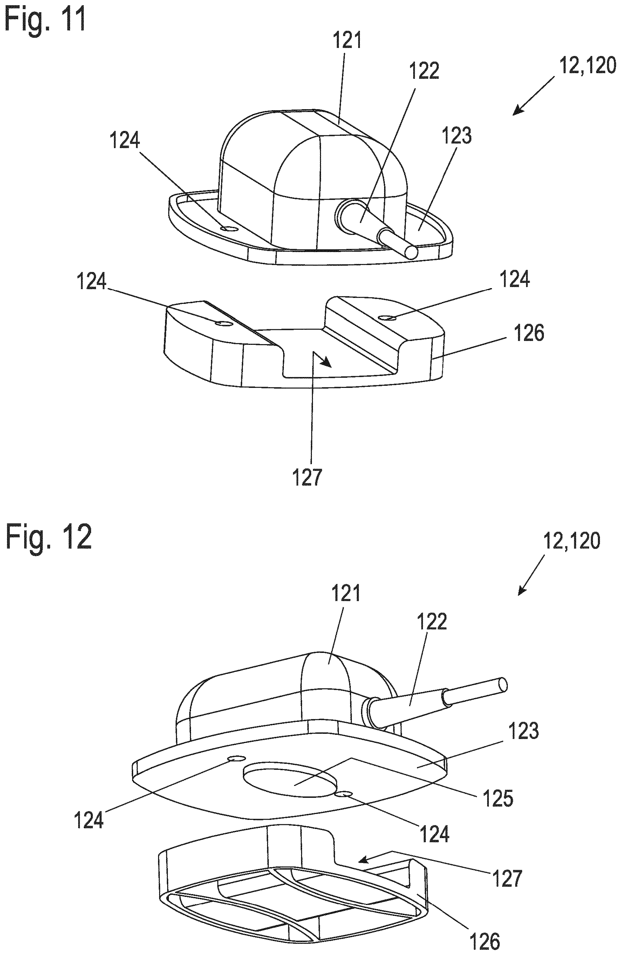

Der beim Ausführungsbeispiel der Fig. 10 eingesetzte Sensor 12 ist in den Figuren 11 und 12 in jeweils einer isometrischen Darstellung aus verschiedenen Blickrichtungen detaillierter dargestellt.

Der verwendete Sensor 12 mit Sensorgehäuse 120 entspricht hier dem bei den Ausführungsbeispielen der Figuren 4 bis 7 verwendeten, der in den Figuren 8 und 9 detaillierter dargestellt ist. Grundsätzlich wäre eine Montage des Sensorgehäuses 120 auch beim Lattenrost des Stützelements 3 gemäß Fig. 10 möglich.

Ein unmittelbares Anschrauben des Sensorgehäuses 120 an eine der Latten ist jedoch nicht vorteilhaft, da die Latten nur eine relativ geringe Stärke von etwa 8-12mm aufweisen und die Gefahr besteht, dass eine eingeschraubte Holzschraube bis durch die Oberseite durchdringt und eine aufgelegte Matratze zerstört und auch eine Verletzungsgefahr birgt. Zudem könnte eine eingeschraubte Holzschraube die Stabilität der Latte beeinträchtigen.

Stattdessen wird der Sensor 120 mit einem Montageadapter 126 an einer der Latten befestigt. Der Montageadapter 125 weist eine der Montageplatte 123 entsprechende Grundform auf, durch die eine Vertiefung 127 läuft, in die eine Latte des Lattenrosts eingesetzt wird. Die Tiefe der Vertiefung 127 ist so gewählt, dass die Latte bei Verschraubung der Montageplatte 123 auf den Montageadapter 126 zwischen diesen beiden eingeklemmt wird und die Erhöhung 125 an der Unterseite des Sensorgehäuses 120 fest auf die entsprechende Latte gedrückt wird.

Der Montageadapter 126 ermöglicht es, den Sensor 12 im Gehäuse 120 sowohl für eine Verwendung mit plattenförmigen Stützelementen als auch bei einem Lattenrost als Stützelement einzusetzen. Für Lattenroste mit unterschiedlich breiten bzw. dicken Latten können unterschiedlich dimensionierte Montageadapter 126 vorgesehen sein.

Das in den Ausführungsbeispielen dieser Anmeldung dargestellte Bett 1 ist jeweils ein Einzelbett. Es wird angemerkt, dass ein anmeldungsgemäßer Möbelantrieb mit dem Sensor 12 auch eingesetzt werden kann, wenn zwei oder mehrere Betten 1 gemäß den vorgenannten Ausführungen zu einer Bettstätte miteinander verbunden werden oder wenn in einem Grundelement mehrere Stützelemente nebeneinander aufgenommen sind. Das Bett weist im letzteren Fall mehrere, bevorzugt zwei, mittlere nicht schwenkbare Abschnitte 3', eine entsprechende Anzahl schwenkbarer Rückenteile 4 und eine entsprechende Anzahl Beinteile 5 auf. Üblicherweise ist jeder Person eine Matratze zugeordnet.

Es können für jedes Einzelbett bzw. jedes Stützelement separate Verstellantriebe vorgesehen sein, die elektrisch gekoppelt sind. Bei mechanisch miteinander verbundenen Einzelbetten bzw. Stützelementen können Schallentkopplungselemente vorgesehen sein, welche die mechanische Verbindung zweier Einzelbetten bzw. Stützelemente ermöglichen, dabei jedoch den durch die Verbindung übertragbaren Körperschall dämpfen oder stark reduzieren.

### Bezugszeichenliste

- 1: Bett
- 2: Grundelement
- 3: Stützelement
- 3': nicht schwenkbarer Abschnitt des Stützelements
- 4: Rückenteil
- 4': erster Abschnitt des Rückenteils
- 4": zweiter Abschnitt des Rückenteils
- 5: Beinteil
- 5': erster Abschnitt des Beinteils
- 5": zweiter Abschnitt des Beinteils
- 6: Bewegungsbeschlag
- 60: Beschlagelement
- 61: Querstütze
- 62: Längsstütze
- 63: Basis
- 64: Schenkel
- 65: Hebel
- 7,8: Verstellantrieb
- 9: Steuereinrichtung
- 10: Handbedienung
- 11: Übertragungsstrecke
- 12: Sensor
- 120: Sensorgehäuse
- 121: kuppelförmiges Gehäuseteil
- 122: Kabeldurchführung
- 123: Montageplatte
- 124: Befestigungsloch
- 125: Erhöhung
- 126: Montageadapter
- 127: Vertiefung
- 13: Sensorkabel
- 14: Mobilgerät
- 15: drahtlose Signale

- 20: Signal
- 21: Herzschlag-Peak
- 22: Einhüllende
- 23: ansteigende Flanke (Einatmung)
- 24: abfallende Flanke (Ausatmung)

- M: Matratze

## Patentansprüche

1. Schlaf- oder Ruhemöbel mit einem elektromotorischen Möbelantrieb, der eine Anzahl Verstellantrieben (7, 8) zur elektromotorischen Bewegung wenigstens eines beweglichen Möbelbauteils relativ zu einem weiteren Möbelbauteil und eine Handbedienung (10) zur Bedienung der Verstellantrieben (7, 8) aufweist, wobei mindestens ein Sensor (12) zur Erfassung von Vibrationen und/oder Schall mit dem Schlaf- oder Ruhemöbel gekoppelt ist und eine mit dem mindestens einen Sensor (12) verbundene Auswerteeinheit vorgesehen ist, und wobei die Auswerteeinheit zur Verarbeitung und Auswertung der Signale des mindestens einen Sensors (12) und zur Erfassung von physiologischen Parametern einer das Schlaf- oder Ruhemöbel benutzenden Person eingerichtet ist, wobei die erfassten physiologischen Parameter eine Herzfrequenz, eine Atemfrequenz und/oder ein Bewegungszustand der Person sind, wobei der mindestens eine Sensor (12) einen piezoelektrisch arbeitenden Schall- bzw. Schwingungsaufnehmer aufweist und die Auswerteeinheit einen Filter, insbesondere einen Tief- oder Bandpassfilter zur Signalverarbeitung aufweist, **dadurch gekennzeichnet, dass**
der Sensor (12) zwischen Befestigungspunkten eines Beschlagelements (60) an dem Rückenteil (4) des Schlaf- oder Ruhemöbels befestigt ist, wobei das Beschlagelement (60) das Rückenteil (4) mit einem der Verstellantriebe (7, 8) koppelt.

2. Schlaf- oder Ruhemöbel nach Anspruch 1, wobei der elektromotorische Möbelantrieb eine Steuereinrichtung (9) zur Ansteuerung der Verstellantriebe (7, 8) aufweist, wobei die Auswerteeinheit mit der Steuereinrichtung (9) gekoppelt ist oder in die Steuereinrichtung (9) integriert ist.

3. Schlaf- oder Ruhemöbel nach einem oder mehreren der vorgenannten Ansprüche, bei dem die Auswerteeinheit einen Speicher zum Abspeichern eines Zeitverlaufs der physiologischen Parameter aufweist.

4. Schlaf- oder Ruhemöbel nach einem oder mehreren der vorgenannten Ansprüche, bei dem die Auswerteeinheit eine Überwachungseinrichtung zum Vergleichen der physiologischen Parameter mit vorgegebenen Grenzwerten aufweist.

5. Schlaf- oder Ruhemöbel nach einem oder mehreren der vorgenannten Ansprüche, bei dem die Auswerteeinheit eine Übertragungseinheit zur Übertragung der physiologischen Parameter auf ein Mobilgerät (14) aufweist.

6. Schlaf- oder Ruhemöbel nach Anspruch 5, bei dem die Übertragungseinheit zur drahtlosen Übertragung der physiologischen Parameter an das Mobilgerät (14) eingerichtet ist, insbesondere über eine WLAN- oder Bluetooth-Übertragungsstrecke.

7. Schlaf- oder Ruhemöbel nach Anspruch 5 oder 6, bei dem das Mobilgerät (14) eine Überwachungseinrichtung zum Vergleichen der physiologischen Parameter mit vorgegebenen Grenzwerten aufweist.

8. Schlaf- oder Ruhemöbel nach einem der Ansprüche 5 bis 7, bei dem das Mobilgerät (14) zusätzlich die Handbedienung (10) darstellt.

9. Schlaf- oder Ruhemöbel nach einem oder mehreren der vorgenannten Ansprüche, bei dem die Auswerteeinheit der Signale des Sensors (12) zusätzlich zur Erfassung und Auswertung von Vibrationen eingerichtet ist, die bei der Betätigung eines oder mehrerer der Verstellantriebe (7, 8) auftreten.

10. Schlaf- oder Ruhemöbel nach Anspruch 9, bei dem die Auswerteeinheit dazu eingerichtet ist, eine Fehlfunktion und/oder eine Überlastung und/oder eine Nicht-Belastung eines oder mehrerer der Verstellantriebe (7, 8) im Betrieb zu ermitteln.

11. Schlaf- oder Ruhemöbel nach einem oder mehreren der vorgenannten Ansprüche, bei dem der Sensor (12) ein Sensorgehäuse (120) mit einer Montagefläche aufweist, auf der eine Erhöhung (125) ausgebildet ist, wobei ein Schall- bzw. Schwingungsaufnehmer des Sensors (12) im Inneren des Sensorgehäuses (120) im Bereich der Erhöhung (125) angeordnet ist.

12. Schlaf- oder Ruhemöbel nach Anspruch 11, bei dem ein Montageadapter (126) vorgesehen ist, der mit dem Sensorgehäuse (120) verbunden wird um eine Latte eines Lattenrosts zur Montage des Sensors (12) an dieser Latte zu umgreifen.

13. Schlaf- oder Ruhemöbel nach einem der Ansprüche 1 bis 12, ausgebildet als Bett (1).

14. Schlaf- oder Ruhemöbel nach Anspruch 13, bei dem der Sensor (12) in einer unteren Hälfte und insbesondere in einem unteren Drittel des Rückenteils (4) des Schlaf- oder Ruhemöbels befestigt ist.

## Claims

1. Piece of sleeping or reclining furniture, having an electromotive furniture drive which comprises a number of adjusting drives (7, 8) for electromotively moving at least one movable furniture component relative to another furniture component and a hand-held operating unit (10) for operating the adjusting drives (7, 8), wherein at least one sensor (12) for detecting vibrations and/or sound is coupled to the piece of sleeping or reclining furniture, and an evaluation unit is provided which is connected to the at least one sensor (12), and wherein the evaluation unit is designed to process and evaluate the signals from the at least one sensor (12) and to detect physiological parameters of a person using the piece of sleeping or reclining furniture, wherein the detected physiological parameters are a heart rate, a respiratory rate and/or a motion state of the person, wherein the at least one sensor (12) has a piezoelectric sound and/or vibration pickup and the evaluation unit has a filter, in particular a low-pass or band-pass filter for signal processing,
**characterized in that**
the sensor (12) is attached to the back part (4) of the piece of sleeping or reclining furniture between attachment points of a fitting element (60), wherein the fitting element (60) couples the back part (4) to one of the adjusting drives (7, 8).

2. Piece of sleeping or reclining furniture according to claim 1, wherein the electromotive furniture drive has a control device (9) for controlling the adjusting drives (7, 8), wherein the evaluation unit is coupled to the control device (9) or is integrated in the control device (9).

3. Piece of sleeping or reclining furniture according to one or more of the preceding claims, in which the evaluation unit has a memory for storing a curve of the physiological parameters over time.

4. Piece of sleeping or reclining furniture according to one or more of the preceding claims, in which the evaluation unit has a monitoring device for comparing the physiological parameters with specified limit values.

5. Piece of sleeping or reclining furniture according to one or more of the preceding claims, in which the evaluation unit has a transmitting unit for transmitting the physiological parameters to a mobile device (14).

6. Piece of sleeping or reclining furniture according to claim 5, in which the transmitting unit is designed to transmit the physiological parameters to the mobile device (14) in a wireless manner, in particular via a WLAN or Bluetooth transmission path.

7. Piece of sleeping or reclining furniture according to claim 5 or 6, in which the mobile device (14) has a monitoring device for comparing the physiological parameters with specified limit values.

8. Piece of sleeping or reclining furniture according to any one of claims 5 to 7, in which the mobile device (14) additionally forms the hand-held operating unit (10).

9. Piece of sleeping or reclining furniture according to one or more of the preceding claims, in which the evaluation unit for the signals from the sensor (12) is additionally designed to detect and evaluate vibrations that occur on actuation of one or more of the adjusting drives (7, 8).

10. Piece of sleeping or reclining furniture according to claim 9, in which the evaluation unit is designed to determine a malfunction and/or an overloading and/or a non-loading of one or more of the adjusting drives (7, 8) during operation.

11. Piece of sleeping or reclining furniture according to one or more of the preceding claims, in which the sensor (12) has a sensor housing (120) with a mounting surface, on which an elevation (125) is formed, wherein a sound and/or vibration pickup of the sensor (12) is arranged in the interior of the sensor housing (120) in the region of the elevation (125).

12. Piece of sleeping or reclining furniture according to claim 11, in which a mounting adapter (126) is provided, which is connected to the sensor housing (120) so as to engage around a slat of a slatted frame in order to mount the sensor (12) on said slat.

13. Piece of sleeping or reclining furniture according to any one of claims 1 to 12, designed as a bed (1).

14. Piece of sleeping or reclining furniture according to claim 13, in which the sensor (12) is attached in a lower half and in particular in a lower third of the back part (4) of the piece of sleeping or reclining furniture.

## Revendications

1. Meuble de couchage ou de repos avec un entraînement pour meuble à moteur électrique présentant un certain nombre d'entraînements de réglage (7, 8) servant au déplacement par moteur électrique au moins d'un élément de meuble mobile par rapport à un autre élément de meuble et une commande manuelle (10) servant à commander les entraînements de réglage (7, 8), au moins un capteur (12) servant à détecter les vibrations et/ou les bruits étant relié au meuble de couchage ou de repos
et une unité d'évaluation étant connectée à au moins un capteur (12) et l'unité d'évaluation étant disposée pour traiter et évaluer les signaux dudit au moins un capteur (12) et pour saisir les paramètres physiologiques d'une personne utilisant le meuble de couchage ou de repos, les paramètres physiologiques étant une fréquence cardiaque, une fréquence respiratoire et/ou un état cinématique de la personne, ledit au moins un capteur (12) présentant un capteur de sons et d'oscillations à fonctionnement piézoélectrique et l'unité d'évaluation présentant un filtre, en particulier un filtre à bande passante basse servant au traitement des signaux, **caractérisé par** ce que
le capteur (12) est fixé entre les points de fixation d'un élément de ferrure (60) sur la partie arrière (4) du meuble de couchage ou de repos, l'élément de ferrure (60) reliant la partie arrière (4) à un des entraînements de réglage (7, 8).

2. Meuble de couchage ou de repos selon la revendication 1, dans lequel l'entraînement pour meuble à moteur électrique présente un dispositif de commande (9) pour commander les entraînements de réglage (7, 8), l'unité d'évaluation étant reliée au dispositif de commande (9) ou étant intégrée dans le dispositif de commande (9).

3. Meuble de couchage et de repos selon une ou plusieurs revendications susmentionnées, dans lequel l'unité d'évaluation présente une mémoire servant à l'enregistrement d'une durée des paramètres physiologiques.

4. Meuble de couchage et de repos selon une ou plusieurs revendications susmentionnées, dans lequel l'unité d'évaluation présente un dispositif de surveillance servant à la comparaison des paramètres physiologiques avec les valeurs limites prédéfinies.

5. Meuble de couchage et de repos selon une ou plusieurs revendications susmentionnées, dans lequel l'unité d'évaluation présente une unité de transmission servant à la transmission des paramètres physiologiques vers un appareil mobile (14).

6. Meuble de couchage ou de repos selon la revendication 5, dans lequel l'unité de transmission est conçue pour la transmission sans fil des paramètres physiologiques vers l'appareil mobile (14), en particulier par voie de transmission Wi-Fi ou Bluetooth.

7. Meuble de couchage ou de repos selon la revendication 5 ou 6, dans lequel l'appareil mobile (14) présente un dispositif de surveillance servant à la comparaison des paramètres physiologiques avec les valeurs limites prédéfinies.

8. Meuble de couchage ou de repos selon l'une des revendications 5 à 7, dans lequel l'appareil mobile (14) est en outre la commande manuelle (10).

9. Meuble de couchage ou de repos selon une ou plusieurs revendications susmentionnées, dans lequel l'unité d'évaluation des signaux du capteur (12) est disposée en plus pour la saisie et l'évaluation des vibrations produites par l'actionnement d'un ou de plusieurs entraînements de réglage (7, 8).

10. Meuble de couchage ou de repos selon la revendication 9, dans lequel l'unité d'évaluation est conçue pour détecter un dysfonctionnement et/ou une surcharge et/ou une absence de charge d'un ou de plusieurs entraînements de réglage (7, 8) pendant le fonctionnement.

11. Meuble de couchage ou de repos selon une ou plusieurs revendications susmentionnées, dans lequel le capteur (12) présente un boîtier de capteur (120) avec une surface de montage, sur laquelle une élévation (125) est réalisée, un capteur de sons et d'oscillations du capteur (12) étant disposé à l'intérieur du boîtier de capteur (120) dans la zone d'élévation (125).

12. Meuble de couchage ou de repos selon la revendication 11, dans lequel un adaptateur de montage (126) est prévu, qui est relié avec le boîtier de capteur (120) afin d'entourer une latte du sommier pour le montage du capteur (12) sur cette latte.

13. Meuble de couchage ou de repos selon l'une des revendications 1 à 12 réalisé comme lit (1).

14. Meuble de couchage ou de repos selon la revendication 13, dans lequel le capteur (12) est fixé dans la moitié inférieure et en particulier dans un tiers inférieur de la partie arrière (4) du meuble de couchage ou de repos.
